# EUROPEAN PATENT APPLICATION

(11) **EP 4 819 033 A1**
(43) Date of publication of application: **07.10.2026**
(21) Application number: 26170881.2
(22) Date of filing: 07.04.2026
(51) Int. Cl.: G06T 5/60, G06T 5/70

(54) **METHOD FOR TRAINING A SIGNAL DENOISING MODEL, SIGNAL DENOISING METHOD, AND DEVICE**

(30) Priority: 03.04.2025 CN 202510423839
(71) Applicant: Shanghai United Imaging Healthcare Co., Ltd., Shanghai 201807 (CN)
(72) Inventor: MAO, Yifu, Shanghai, 201807 (CN)
(74) Representative: Murgitroyd & Company

(57) **Abstract**

The present application relates to a method for training a signal denoising model, a signal denoising method, and a device. According to the present application, the method includes: performing stage-by-stage denoising training on a multi-stage network based on training signal data corresponding to the multi-stage network, to obtain a signal denoising model, obtaining, when a current stage subjected to the stage-by-stage denoising training is not a first stage, a loss value of a current-stage network based on training signal data corresponding to the current-stage network, and after gradient descent is performed individually on the current-stage network based on the loss value, performing, based on the loss value, gradient descent jointly on current multiple stages of the network that have undergone the gradient descent individually.

## Description

### TECHNICAL FIELD

The present application relates to the field of signal processing technologies, and in particular to a method for training a signal denoising model, a signal denoising method, and a computer device.

### BACKGROUND

A signal is a physical quantity carrying information. Most signals carry a certain amount of noise, which is a type of interference information. Noise adversely affects signal processing and reduces signal quality and intelligibility.

Denoising a signal can reduce noise in the signal, improve the signal-to-noise ratio, and prevent the signal from being overwhelmed by excessive noise. Conventional techniques can perform denoising using a trained model. However, the denoising effect of such models is sometimes unsatisfactory.

### SUMMARY

In view of the above technical problems, it is necessary to provide a method and apparatus for training a signal denoising model, a signal denoising method and apparatus, a computer device, a storage medium, and a computer program product.

In a first aspect, a method for training a signal denoising model is provided in the present application. The method includes: performing stage-by-stage denoising training on a multi-stage network based on training signal data corresponding to the multi-stage network, to obtain a signal denoising model; obtaining, when a current stage subjected to the stage-by-stage denoising training is not a first stage, a loss value of a current-stage network based on training signal data corresponding to the current-stage network; and after gradient descent is performed individually on the current-stage network based on the loss value, performing, based on the loss value, gradient descent jointly on current multiple stages of the network that have undergone the gradient descent individually.

In a second aspect, an apparatus for training a signal denoising model is provided in the present application. The apparatus includes: a model acquisition module configured to perform stage-by-stage denoising training on a multi-stage network based on training signal data corresponding to the multi-stage network, to obtain a signal denoising model; and a training module configured to obtain, when a current stage subjected to the stage-by-stage denoising training is not a first stage, a loss value of a current-stage network based on training signal data corresponding to the current-stage network, and after gradient descent is performed individually on the current-stage network based on the loss value, perform, based on the loss value, gradient descent jointly on current multiple stages of the network that have undergone the gradient descent individually.

In a third aspect, a signal denoising method is provided in the present application. The method includes: obtaining a to-be-denoised signal; and inputting the to-be-denoised signal into a signal denoising model to obtain a denoised signal, where the signal denoising model is trained by using a method for training a signal denoising model according to the first aspect.

In a fourth aspect, a signal denoising apparatus is provided in the present application. The apparatus includes: a signal acquisition module configured to obtain a to-be-denoised signal; and a denoising module configured to input the to-be-denoised signal into a signal denoising model to obtain a denoised signal. The signal denoising model is trained by using the method for training a signal denoising model according to the first aspect.

In a fifth aspect, a computer device is provided in the present application, including a memory and a processor. The memory stores a computer program. The processor, when executing the computer program, implements the steps of the method according to the first aspect or the third aspect.

In a sixth aspect, a computer-readable storage medium is provided in the present application, having a computer program stored thereon. The computer program, when executed by a processor, causes the processor to implement the steps of the method according to the first aspect or the third aspect.

In a seventh aspect, a computer program product having a computer program stored thereon is provided in the present application. The computer program, when executed by a processor, implements steps of the method according to the first aspect or the third aspect.

Details of one or more embodiments of the present application are set forth in the accompanying drawings and the description below. Other features, objectives, and advantages of the present application will become apparent from the description, the accompanying drawings, and the claims.

### BRIEF DESCRIPTION OF THE DRAWINGS

To describe the technical solutions in the embodiments of the present application or in the related art more clearly, the following briefly introduces the accompanying drawings required for describing the embodiments or the related art. Apparently, the accompanying drawings in the following description are only some embodiments of the present application, and those of ordinary skill in the art can also obtain other accompanying drawings according to these accompanying drawings without creative efforts.
FIG. 1 is a flow diagram of a method for training a signal denoising model according to an embodiment.
FIG. 2(a) is a schematic diagram of series connection of a four-stage network according to an embodiment.
FIG. 2(b) is a schematic diagram of a configuration of first-stage denoising training according to an embodiment.
FIG. 2(c) is a schematic diagram of a configuration of second-stage denoising training according to an embodiment.
FIG. 2(d) is a schematic diagram of a configuration of third-stage denoising training according to an embodiment.
FIG. 2(e) is a schematic diagram of a configuration of fourth-stage denoising training according to an embodiment.
FIG. 3(a) is a schematic diagram of a configuration of first-stage denoising training with CT multi-energy-level data according to an embodiment.
FIG. 3(b) is a schematic diagram of a configuration of second-stage denoising training with CT multi-energy-level data according to an embodiment.
FIG. 4 is a flow diagram of a signal denoising method according to an embodiment.
FIG. 5 is a flow diagram of an inference phase according to an embodiment.
FIG. 6 is a schematic diagram of an internal configuration of a computer device according to an embodiment.

### DETAILED DESCRIPTION OF THE EMBODIMENTS

In order to make the objectives, technical solutions, and advantages of the present application clearer and more explicit, the present application is further described in detail below with reference to the accompanying drawings and embodiments. It should be understood that the specific embodiments described herein are merely used to explain the present application and are not intended to limit the present application.

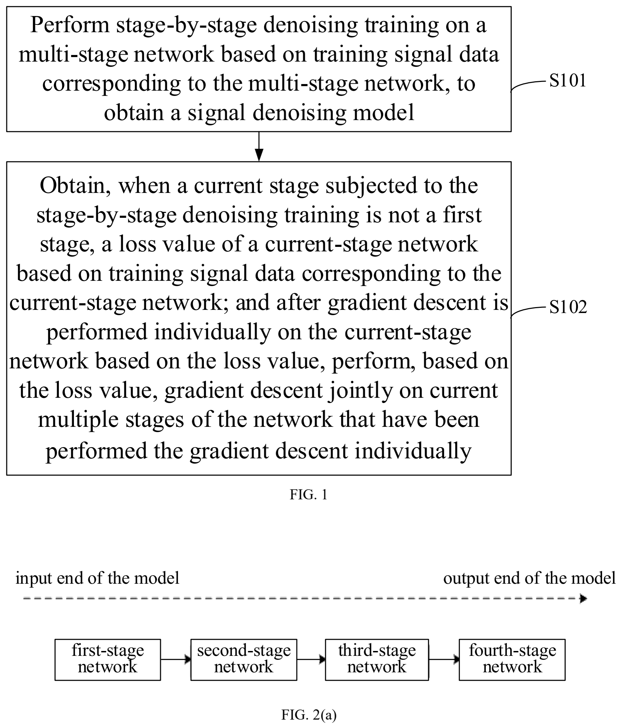

The method for training a signal denoising model and the signal denoising method provided in the present application may be executed by a computer device. The method for training a signal denoising model may include steps shown in FIG. 1.

In step S101, stage-by-stage denoising training is performed on a multi-stage network based on training signal data corresponding to the multi-stage network, to obtain a signal denoising model.

When constructing a signal denoising model, the number of networks included in the signal denoising model may be determined according to actual requirements, and the number of networks may be set to be greater than or equal to 2.

A plurality of networks may be connected in series. Correspondingly, a level relationship exists between the networks. A network closer to an input end of the model has a smaller level. A network closest to the input end of the model may be referred to as a first-stage network, and a network closest to an output end of the model may be referred to as a last-stage network. Assuming that the number of networks is an integer n, description is made by taking n equal to 4 as an example. As shown in FIG. 2(a), in a four-stage network, the first-stage network is closest to the input end of the model, and a fourth-stage network is closest to the output end of the model.

A corresponding denoising range is calibrated for each stage network, i.e., a corresponding denoising calibration range is set for each stage network. A network closer to the input end of the model (i.e., a network with a smaller level) has a larger denoising calibration range, and a network closer to the output end of the model (i.e., a network with a larger level) has a smaller denoising calibration range.

In an order from a smaller level to a larger level of networks, denoising calibration ranges corresponding to the multiple stages of the network decrease sequentially and present a decreasing relationship. In addition, the denoising calibration ranges corresponding to the multiple stages of the network may be independent of each other, and the denoising calibration ranges corresponding to the multiple stages of the network may jointly form a larger range.

A denoising calibration range may be defined by an absolute noise level (i.e., magnitude) or a relative noise level. The absolute noise level is an actual physical measure of noise and is a direct measure of the magnitude of noise. The relative noise level is a comparison value of noise relative to specific noise and may be expressed in a percentage form.

Assuming that the number of networks is n, description is made by taking n equal to 4 as an example. When the denoising calibration range is divided by the absolute noise level, the denoising calibration range corresponding to the first-stage network may be 400 to 300, the denoising calibration range corresponding to the second-stage network may be 300 to 200, the denoising calibration range corresponding to the third-stage network may be 200 to 100, and the denoising calibration range corresponding to the fourth-stage network may be 100 to 0. The denoising calibration ranges corresponding to the four stages of networks decrease sequentially in the order from the smaller level to the larger level of networks, i.e., upper limit values or lower limit values of the denoising calibration ranges corresponding to the four stages of the networks decrease in the order from the smaller network level to the larger network level. The denoising calibration ranges may be independent of each other, and the larger range jointly formed by the denoising calibration ranges may be 400 to 0.

When the denoising calibration range is divided by the relative noise level, the denoising calibration range corresponding to the first-stage network may be 100% to 75%, the denoising calibration range corresponding to the second-stage network may be 75% to 50%, the denoising calibration range corresponding to the third-stage network may be 50% to 25%, and the denoising calibration range corresponding to the fourth-stage network may be 25% to 0. The denoising calibration ranges corresponding to the four stages of networks decrease sequentially in the order from the smaller level to the larger level of networks, the denoising calibration ranges may be independent of each other, and the larger range jointly formed by the denoising calibration ranges may be 100% to 0.

Span magnitudes of the denoising calibration ranges corresponding to the multiple stages of the network may be the same or different. A span of a denoising calibration range corresponding to a single-stage network is obtained by subtracting a lower limit value of the denoising calibration range corresponding to the single-stage network from an upper limit value of the denoising calibration range corresponding to the single-stage network.

When the span magnitudes of the denoising calibration ranges corresponding to the multiple stages of the network are the same, the denoising calibration ranges corresponding to the multiple stages of the network decrease in equal proportions. When the spans of the denoising calibration ranges corresponding to the multiple stages of the network are different, the denoising calibration ranges corresponding to the multiple stages of the network may decrease based on a logarithmic function.

When the denoising calibration range is divided by the absolute noise level, in some situations, the denoising calibration range corresponding to the first-stage network is 400 to 300, the denoising calibration range corresponding to the second-stage network is 300 to 200, the denoising calibration range corresponding to the third-stage network is 200 to 100, and the denoising calibration range corresponding to the fourth-stage network is 100 to 0. In this situation, the span magnitudes of the denoising calibration ranges corresponding to the multiple stages of the network are the same, which are all 100.

When the denoising calibration range is divided by the absolute noise level, in other situations, the denoising calibration range corresponding to the first-stage network is 400 to 350, the denoising calibration range corresponding to the second-stage network is 350 to 250, the denoising calibration range corresponding to the third-stage network is 250 to 100, and the denoising calibration range corresponding to the fourth-stage network is 100 to 0. In this situation, the span magnitudes of the denoising calibration ranges corresponding to the multiple stages of the network are different, the span of the denoising calibration range corresponding to the first-stage network is 50, the span of the denoising calibration range corresponding to the second-stage network is 100, the span of the denoising calibration range corresponding to the third-stage network is 150, and the span of the denoising calibration range corresponding to the fourth-stage network is 100.

When the denoising calibration range is divided by the relative noise level, in some situations, the denoising calibration range corresponding to the first-stage network is 100% to 75%, the denoising calibration range corresponding to the second-stage network is 75% to 50%, the denoising calibration range corresponding to the third-stage network is 50% to 25%, and the denoising calibration range corresponding to the fourth-stage network is 25% to 0. In this situation, the span magnitudes of the denoising calibration ranges corresponding to the multiple stages of the network are the same, which are all 25%.

When the denoising calibration range is divided by the relative noise level, in other situations, the denoising calibration range corresponding to the first-stage network is 100% to 85%, the denoising calibration range corresponding to the second-stage network is 85% to 60%, the denoising calibration range corresponding to the third-stage network is 60% to 25%, and the denoising calibration range corresponding to the fourth-stage network is 25% to 0. In this situation, the span magnitudes of the denoising calibration ranges corresponding to the multiple stages of the network are different, the span of the denoising calibration range corresponding to the first-stage network is 15%, the span of the denoising calibration range corresponding to the second-stage network is 25%, the span of the denoising calibration range corresponding to the third-stage network is 35%, and the span of the denoising calibration range corresponding to the fourth-stage network is 25%.

The denoising calibration range corresponding to each stage network is configured to calibrate that each stage network can reduce the noise carried by a signal from a certain magnitude to another magnitude. The noise level before reduction corresponds to the upper limit value of the denoising calibration range, and the noise level after reduction corresponds to the lower limit value of the denoising calibration range.

Therefore, after the denoising calibration range corresponding to each stage network is determined, the noise level carried by training signal data input into each stage network and the noise level to be reduced may be determined based on the denoising calibration range of each stage network. Training signal data (which may be referred to as training input signal data) as an input and having a corresponding noise level is constructed based on the noise level carried by the training signal data input into each stage network. Training signal data (which may be referred to as training target signal data) as a target and having a corresponding noise level is constructed based on the noise level to be reduced. The training input signal data and the training target signal data form a set of training signal data.

It may be understood that each stage network may correspond to at least one set of training signal data.

After the training signal data corresponding to the multi-stage network are obtained, stage-by-stage denoising training may be performed on the multi-stage network based on the training signal data in the order from the smaller level to the larger level of networks. The first-stage network is trained first, then the second-stage network is trained, and so on, and finally the last-stage network is trained. After all stages of networks are trained, one iteration is deemed to be completed. A plurality of iterations may be performed according to actual requirements.

A related training process of a single iteration is described below.

When a current stage subjected to stage-by-stage denoising training is a first stage, i.e., first-stage denoising training is currently performed, a loss value of the first-stage network may be obtained based on training signal data corresponding to the first-stage network. Based on the loss value of the first-stage network, gradient descent is performed on the first-stage network individually. At this time, the first-stage denoising training is completed, and next-stage denoising training may be performed.

In step S102, when a current stage subjected to the stage-by-stage denoising training is not a first stage, a loss value of a current-stage network is obtained based on training signal data corresponding to the current-stage network. After gradient descent is performed individually on the current-stage network based on the loss value, gradient descent is performed, based on the loss value, jointly on current multiple stages of the network that have undergone the gradient descent individually.

For example, when the current stage subjected to stage-by-stage denoising training is the second stage, i.e., second-stage denoising training is currently performed, a loss value of the second-stage network may be obtained based on training signal data corresponding to the second-stage network. Based on the loss value of the second-stage network, gradient descent is performed individually on the second-stage network. After the gradient descent is performed individually on the second-stage network, at this time, the current multiple stages of the network that have undergone the gradient descent individually include the second-stage network and networks with levels lower than the second stage (i.e., the first-stage network). Gradient descent may be performed jointly on the first-stage network and the second-stage network based on the loss value of the second-stage network. At this time, the second-stage denoising training is completed, and next-stage denoising training may be performed.

When the current stage subjected to stage-by-stage denoising training is the third stage, i.e., third-stage denoising training is currently performed, a loss value of the third-stage network may be obtained based on training signal data corresponding to the third-stage network. Based on the loss value of the third-stage network, gradient descent is performed individually on the third-stage network. After the gradient descent is performed individually on the third-stage network, at this time, the current multiple stages of the network that have undergone the gradient descent individually include the third-stage network and networks with levels lower than the third stage (i.e., the first-stage network and the second-stage network). The gradient descent may be performed jointly on the first-stage network, the second-stage network, and the third-stage network based on the loss value of the third-stage network. At this time, the third-stage denoising training is completed, and next-stage denoising training may be performed.

Denoising training of remaining stages is performed according to the method described above until last-stage denoising training is completed. After the last-stage denoising training is completed, one iteration may be deemed to be completed.

In the above-mentioned method for training a signal denoising model, stage-by-stage denoising training is performed on a multi-stage network based on training signal data corresponding to the multi-stage network, so as to obtain a signal denoising model. When a current stage subjected to stage-by-stage denoising training is not a first stage, a loss value of a current-stage network is obtained based on training signal data corresponding to the current-stage network. After gradient descent is performed individually on the current-stage network based on the loss value, gradient descent is performed, based on the loss value, jointly on current multiple stages of the network that have undergone the gradient descent individually. In the present application, a stage-by-stage denoising method is adopted to reduce part of noise for each stage network. In a training process, gradient descent is performed not only on a single-stage network individually but also on a multi-stage network jointly, which can improve denoising capability of the signal denoising model and enhance a model denoising effect.

In some embodiments, the training signal data corresponding to the current-stage network are added with a part of noise signals removed by a previous-stage network of the current-stage network.

For example, when the current stage subjected to stage-by-stage denoising training is a first stage, i.e., first-stage denoising training is currently performed, a loss value of the first-stage network may be obtained based on training signal data corresponding to the first-stage network. Based on the loss value of the first-stage network, gradient descent is performed individually on the first-stage network. At this time, the first-stage denoising training is completed, and next-stage denoising training may be performed.

In an embodiment, when the current stage subjected to stage-by-stage denoising training is the second stage, i.e., second-stage denoising training is currently performed, a part of the noise signals removed by the first-stage network may be added to training signal data corresponding to the second-stage network, and a loss value of the second-stage network is obtained based on the training signal data corresponding to the second-stage network that are added with the signals. Based on the loss value of the second-stage network, gradient descent is performed individually on the second-stage network. After the gradient descent is performed individually on the second-stage network, at this time, the current multiple stages of the network that have undergone the gradient descent individually include the second-stage network and networks with levels lower than the second stage (i.e., the first-stage network). The gradient descent may be performed jointly on the first-stage network and the second-stage network based on the loss value of the second-stage network. At this time, the second-stage denoising training is completed, and next-stage denoising training may be performed.

When the current stage subjected to stage-by-stage denoising training is the third stage, i.e., third-stage denoising training is currently performed, the noise signals removed by the second-stage network may be added to training signal data corresponding to the third-stage network, and a loss value of the third-stage network is obtained based on the training signal data corresponding to the third-stage network that are added with the signals. Based on the loss value of the third-stage network, gradient descent is performed individually on the third-stage network. After the gradient descent is performed individually on the third-stage network, at this time, the current multiple stages of the network that have undergone the gradient descent individually include the third-stage network and networks with levels lower than the third stage (i.e., the first-stage network and the second-stage network). The gradient descent may be performed jointly on the first-stage network, the second-stage network, and the third-stage network based on the loss value of the third-stage network. At this time, the third-stage denoising training is completed, and next-stage denoising training may be performed.

Denoising training of remaining stages is performed according to the method described above until last-stage denoising training is completed. After the last-stage denoising training is completed, one iteration may be deemed to be completed.

When a part of noise signals removed by a previous-stage network of a current-stage network are added to training signal data corresponding to the current-stage network, specifically, the noise signals removed by the previous-stage network of the current-stage network in a determined proportion may be added to the training signal data corresponding to the current-stage network. The determined proportion may be set according to actual requirements, and the determined proportion may be between 0 and 1. The determined proportions used in denoising training at different levels may be the same or different.

In a situation where the determined proportions used in denoising training at different levels are the same, for example, 80% of the noise signals removed by the first-stage network are added to training signal data corresponding to the second-stage network, and 80% of the noise signals removed by the second-stage network are added to training signal data corresponding to the third-stage network.

In a situation where the determined proportions used in denoising training at different levels are different, for example, 90% of the noise signals removed by the first-stage network are added to training signal data corresponding to the second-stage network, and 80% of the noise signals removed by the second-stage network are added to training signal data corresponding to the third-stage network.

In the present embodiment, in a stage-by-stage denoising training process, a part of noise signals removed by a previous-stage network are added to training signal data corresponding to a current-stage network to compensate and restore signals, so as to avoid a situation that signals are weakened after denoising by each stage network in the training process, so that the signal denoising model can perform denoising while ensuring signal accuracy. Especially when the signal denoising model processes some signals carrying relatively large noise, the situation that signals are weakened can be relieved apparently.

In some embodiments, performing gradient descent individually on the current-stage network based on the loss value includes: performing layer-by-layer backward calculation on the current-stage network based on the loss value to obtain a first type of gradient values, and performing gradient descent on the current-stage network based on the first type of gradient values.

Each stage network may include multiple layers. Therefore, when gradient descent is performed individually on a single-stage network, layer-by-layer backward calculation may be performed on the single-stage network to obtain gradient values. A direction corresponding to layer-by-layer backward calculation is from a layer closest to the output end of the model to a layer closest to the input end of the model. The gradient values obtained by layer-by-layer backward calculation are mainly used for individual gradient descent. For distinction, the gradient values obtained by layer-by-layer backward calculation may be referred to as first type of gradient values.

Description is made by taking current performance of first-stage denoising training as an example. A loss value of the first-stage network is obtained based on training signal data corresponding to the first-stage network. Based on the loss value of the first-stage network, layer-by-layer backward calculation is performed on the first-stage network to obtain first type of gradient values. Based on the first type of gradient values, gradient descent is performed on the first-stage network to update parameters in the first-stage network.

In the present embodiment, layer-by-layer backward calculation is performed on a current-stage network based on a loss value of the current-stage network to obtain first type of gradient values, and gradient descent is performed on the current-stage network based on the first type of gradient values, so that parameters in the current-stage network can be updated in a direction in which a loss function value becomes smaller.

In some embodiments, performing, based on the loss value, gradient descent jointly on the current multiple stages of the network that have undergone the gradient descent individually includes: performing, based on the loss value, network-by-network backward calculation on the current multiple stages of the network that have undergone the gradient descent individually to obtain a second type of gradient values, and performing, based on the second type of gradient values, gradient descent on the current multiple stages of the network that have undergone the gradient descent individually.

During joint gradient descent, the number of stages of the current networks that have undergone the gradient descend individually is greater than or equal to two. Network-by-network backward calculation is performed on the current multiple stages of the network that have undergone the gradient descent individually to obtain gradient values. A direction corresponding to network-by-network backward calculation is from a network closest to the output end of the model to a network closest to the input end of the model. The gradient values obtained by network-by-network backward calculation are mainly used for joint gradient descent. For distinction, the gradient values obtained by network-by-network backward calculation may be referred to as a second type of gradient values.

Description is made by taking current performance of second-stage denoising training as an example. At this time, the current multiple stages of the network that have undergone the gradient descent individually include a first-stage network and a second-stage network. Based on a loss value of the second-stage network, network-by-network backward calculation is performed on the first-stage network and the second-stage network to obtain a second type of gradient values. Based on the second type of gradient values, gradient descent is performed on the first-stage network and the second-stage network.

Description is made by taking current performance of third-stage denoising training as an example. At this time, the current multiple stages of the network that have undergone the gradient descent individually include a first-stage network, a second-stage network, and a third-stage network. Based on a loss value of the third-stage network, network-by-network backward calculation is performed on the first-stage network, the second-stage network, and the third-stage network to obtain a second type of gradient values. Based on the second type of gradient values, gradient descent is performed on the first-stage network, the second-stage network, and the third-stage network.

In the present embodiment, based on a loss value of a current-stage network, network-by-network backward calculation is performed, on current multiple stages of the network that have undergone the gradient descent individually to obtain the second type of gradient values, and based on the second type of gradient values, gradient descent is performed on the current multiple stages of the network that have undergone the gradient descent individually, so that parameters in the current multiple stages of the network that have undergone the gradient descent individually can be updated in a direction in which a loss function value becomes smaller. By means of joint gradient descent, convergence to the optimal solution can be achieved more quickly.

In some embodiments, the method for training a signal denoising model provided in the present application further includes: obtaining training signal data corresponding to each stage network based on a denoising calibration range corresponding to each stage network. Each piece of training signal data includes training input signal data and training target signal data. A noise level carried in the training input signal data is an upper limit value of the denoising calibration range corresponding to a respective stage network, and a noise level carried in the training target signal data is a lower limit value of the denoising calibration range corresponding to the respective stage network.

Obtaining the loss value of a current-stage network based on training signal data corresponding to the current-stage network includes: inputting training input signal data corresponding to the current-stage network into the current-stage network; and obtaining the loss value of the current-stage network based on a difference in noise level between an output result of the current-stage network and the training target signal data corresponding to the current-stage network.

The denoising calibration range corresponding to each stage network is configured to calibrate that each stage network can reduce the noise carried by a signal from a certain magnitude to another magnitude. The noise level before reduction corresponds to the upper limit value of the denoising calibration range, and the noise level after reduction corresponds to the lower limit value of the denoising calibration range.

Therefore, after the denoising calibration range corresponding to each stage network is determined, the noise level carried by training signal data input into each stage network is determined based on the denoising calibration range of each stage network, and then training input signal data having a corresponding noise level is constructed. The noise level to be reduced is determined based on the denoising calibration range of each stage network, and then training target signal data having a corresponding noise level is constructed. The training input signal data and the training target signal data form a set of training signal data.

In a situation where the denoising calibration range is divided by the absolute noise level, description is made by taking a first-stage network with a denoising calibration range of 400 to 300 as an example. The upper limit value of the denoising calibration range corresponding to the first-stage network is 400, and training input signal data carrying a noise level of 400 may be constructed. The lower limit value of the denoising calibration range corresponding to the first-stage network is 300, and training target signal data carrying a noise level of 300 may be constructed.

In some situations, predetermined noise-free signals may be obtained. For any stage network, noise with a magnitude equal to an upper limit value of a denoising calibration range corresponding to the stage network is added to the noise-free signals to obtain training input signal data corresponding to the stage network, and noise with a magnitude equal to a lower limit value of the denoising calibration range corresponding to the stage network is added to the noise-free signals to obtain training target signal data corresponding to the stage network.

Description is made by taking a first-stage network with a denoising calibration range of 400 to 300 as an example. After noise-free signals are obtained, noise with a magnitude of 400 may be simulated and generated, and the noise is added to the noise-free signals, so as to obtain training input signal data carrying an absolute noise level of 400. Noise with a magnitude of 300 may also be simulated and generated, and the noise is added to the noise-free signals, so as to obtain training target signal data carrying an absolute noise level of 300.

In other situations, predetermined noisy signals may be obtained and a noise level of the noisy signals is determined. For any stage network, a relative magnitude relationship between the noise level of the noisy signals and an upper limit value of a denoising calibration range may be determined, and a relative magnitude relationship between the noise level of the noisy signals and a lower limit value of the denoising calibration range may be determined.

When the noise of the noisy signals is greater than the upper limit value of the denoising calibration range and the noise of the noisy signals is greater than the lower limit value of the denoising calibration range, the noise level of the noisy signals may be reduced to the upper limit value of the denoising calibration range by means of a predetermined denoising algorithm, so as to obtain training input signal data corresponding to the stage network. The noise level of the noisy signals is reduced to the lower limit value of the denoising calibration range by means of the denoising algorithm, so as to obtain training target signal data corresponding to the stage network.

When the noise of the noisy signals is less than the upper limit value of the denoising calibration range and the noise of the noisy signals is greater than the lower limit value of the denoising calibration range, noise may be added to the noisy signals, so that the noise level of the noisy signals is increased to the upper limit value of the denoising calibration range, so as to obtain training input signal data corresponding to the stage network. The noise level of the noisy signals is reduced to the lower limit value of the denoising calibration range by means of the denoising algorithm, so as to obtain training target signal data corresponding to the stage network.

When the noise of the noisy signals is less than the upper limit value of the denoising calibration range and the noise of the noisy signals is less than the lower limit value of the denoising calibration range, noise may be added to the noisy signals, so that the noise level of the noisy signals is increased to the upper limit value of the denoising calibration range, so as to obtain training input signal data corresponding to the stage network. Noise is added to the noisy signals, so that the noise level of the noisy signals is increased to the lower limit value of the denoising calibration range, so as to obtain training target signal data corresponding to the stage network.

When the noise of the noisy signals is equal to the upper limit value of the denoising calibration range, the noisy signals may be directly used as training input signal data corresponding to the stage network.

When the noise of the noisy signals is equal to the lower limit value of the denoising calibration range, the noisy signals may be directly used as training target signal data corresponding to the stage network.

The predetermined denoising algorithm may include, but is not limited to, a low-pass filtering algorithm, an iterative denoising algorithm, a deep learning denoising algorithm, and the like.

Description is made by taking a first-stage network with a denoising calibration range of 400 to 300 as an example. In the method described above, training input signal data carrying an absolute noise level of 400 corresponding to the first-stage network may be obtained, and training target signal data carrying an absolute noise level of 300 corresponding to the first-stage network may be obtained. Then, the training input signal data carrying the absolute noise level of 400 corresponding to the first-stage network may be input into the first-stage network to obtain an output result of the first-stage network. A loss value of the first-stage network may be obtained based on a difference in noise level between the output result of the first-stage network and the training target signal data carrying the absolute noise level of 300 corresponding to the first-stage network. Subsequently, individual gradient descent and joint gradient descent may be performed based on the loss value of the first-stage network.

Description is made by taking a second-stage network with a denoising calibration range of 300 to 200 as an example. In the method described above, training input signal data carrying an absolute noise level of 300 corresponding to the second-stage network may be obtained, and training target signal data carrying an absolute noise level of 200 corresponding to the second-stage network may be obtained. Then, the training input signal data corresponding to the second-stage network and carrying the absolute noise level of 300 may be input into the second-stage network to obtain an output result of the second-stage network. A loss value of the second-stage network may be obtained based on a difference in noise level between the output result of the second-stage network and the training target signal data corresponding to the second-stage network and carrying the absolute noise level of 200. Subsequently, individual gradient descent and joint gradient descent may be performed based on the loss value of the second-stage network.

In a situation where the denoising calibration range is divided by the relative noise level, description is made by taking a first-stage network with a denoising calibration range of 100% to 75% as an example. In this situation, a noise level may be calibrated first, and the noise level is referred to as a calibrated noise value. Description is made by taking a calibrated noise value of 500 as an example.

Since the upper limit value of the denoising calibration range corresponding to the first-stage network is 100%, a product of the calibrated noise value and the upper limit value is 500, and training input signal data carrying a noise level of 500 may be constructed. Since the lower limit value of the denoising calibration range corresponding to the first-stage network is 75%, a product of the calibrated noise value and the lower limit value is 375, and training target signal data carrying a noise level of 375 may be constructed.

In some situations, predetermined noise-free signals may be obtained. For any stage network, a product (referred to as a first product) of an upper limit value of a denoising calibration range corresponding to the stage network and a calibrated noise value may be obtained, and a product (referred to as a second product) of a lower limit value of the denoising calibration range corresponding to the stage network and the calibrated noise value may be obtained. Noise with a magnitude corresponding to the first product is added to the noise-free signals to obtain training input signal data corresponding to the stage network, and noise with a magnitude corresponding to the second product is added to the noise-free signals to obtain training target signal data corresponding to the stage network.

Description is made by taking a first-stage network with a denoising calibration range of 100% to 75% and a calibrated noise value of 500 as an example. A first product of the upper limit value of the denoising calibration range corresponding to the first-stage network and the calibrated noise value is 500, and a first product of the lower limit value of the denoising calibration range corresponding to the first-stage network and the calibrated noise value is 375. After noise-free signals are obtained, noise with a magnitude of 500 may be simulated and generated, and the noise is added to the noise-free signals, so as to obtain training input signal data carrying a noise level of 500. Noise with a magnitude of 375 may also be simulated and generated, and the noise is added to the noise-free signals, so as to obtain training target signal data carrying a noise level of 375.

In other situations, predetermined noisy signals may be obtained and a noise level of the noisy signals is determined. For any stage network, a relative magnitude relationship between the noise level of the noisy signals and the first product may be determined, and a relative magnitude relationship between the noise level of the noisy signals and the second product may be determined.

When the noise of the noisy signals is greater than the first product and the noise of the noisy signals is greater than the second product, the noise level of the noisy signals may be reduced to a numerical magnitude corresponding to the first product by means of a predetermined denoising algorithm, so as to obtain training input signal data corresponding to the stage network. The noise level of the noisy signals is reduced to a numerical magnitude corresponding to the second product by means of the denoising algorithm, so as to obtain training target signal data corresponding to the stage network.

When the noise of the noisy signals is less than the first product and the noise of the noisy signals is greater than the second product, noise may be added to the noisy signals, so that the noise level of the noisy signals is increased to a numerical magnitude corresponding to the first product, so as to obtain training input signal data corresponding to the stage network. The noise level of the noisy signals is reduced to a numerical magnitude corresponding to the second product by means of the denoising algorithm, so as to obtain training target signal data corresponding to the stage network.

When the noise of the noisy signals is less than the first product and the noise of the noisy signals is less than the second product, noise may be added to the noisy signals, so that the noise level of the noisy signals is increased to a numerical magnitude corresponding to the first product, so as to obtain training input signal data corresponding to the stage network. Noise is added to the noisy signals, so that the noise level of the noisy signals is increased to a numerical magnitude corresponding to the second product, so as to obtain training target signal data corresponding to the stage network.

When the noise of the noisy signals is equal to the first product, the noisy signals may be directly used as training input signal data corresponding to the stage network.

When the noise of the noisy signals is equal to the second product, the noisy signals may be directly used as training target signal data corresponding to the stage network.

The predetermined denoising algorithm may include, but is not limited to, a low-pass filtering algorithm, an iterative denoising algorithm, a deep learning denoising algorithm, and the like.

Description is made by taking a first-stage network with a denoising calibration range of 100% to 75% as an example. In the method described above, training input signal data corresponding to the first-stage network and carrying a relative noise level of 100% may be obtained, and training target signal data corresponding to the first-stage network and carrying a relative noise level of 75% may be obtained. Then, the training input signal data corresponding to the first-stage network and carrying the relative noise level of 100% may be input into the first-stage network to obtain an output result of the first-stage network. A loss value of the first-stage network may be obtained based on a difference in noise level between the output result of the first-stage network and the training target signal data corresponding to the first-stage network and carrying the relative noise level of 75%. Subsequently, individual gradient descent and joint gradient descent may be performed based on the loss value of the first-stage network.

Description is made by taking a second-stage network with a denoising calibration range of 75% to 50% as an example. In the method described above, training input signal data corresponding to the second-stage network and carrying a relative noise level of 75% may be obtained, and training target signal data corresponding to the second-stage network and carrying a relative noise level of 50% may be obtained. Then, the training input signal data corresponding to the second-stage network and carrying the relative noise level of 75% may be input into the second-stage network to obtain an output result of the second-stage network. A loss value of the second-stage network may be obtained based on a difference in noise level between the output result of the second-stage network and the training target signal data corresponding to the second-stage network and carrying the relative noise level of 50%. Subsequently, individual gradient descent and joint gradient descent may be performed based on the loss value of the second-stage network.

In some embodiments, before inputting training input signal data corresponding to a current-stage network into the current-stage network, the method for training a signal denoising model provided in the present application further includes: obtaining noise removed by a previous-stage network of the current-stage network based on training input signal data and training target signal data corresponding to the previous-stage network, performing signal extraction on the noise removed by the previous-stage network of the current-stage network, and adding a part of extracted signals to the training input signal data corresponding to the current-stage network.

Description is made by taking current performance of second-stage denoising training and division of a denoising calibration range by an absolute noise level as an example. When a denoising calibration range of a second-stage network is 300 to 200, training input signal data carrying an absolute noise level of 300 corresponding to the second-stage network and training target signal data carrying an absolute noise level of 200 corresponding to the second-stage network may be obtained. Correspondingly, a previous-stage network of the second-stage network is a first-stage network.

Difference processing may be performed on training input signal data and training target signal data corresponding to the first-stage network to obtain difference signal data. The difference signal data are not greatly different from noise actually removed by the first-stage network. Therefore, the difference signal data may be used as the noise removed by the first-stage network. Then, signal extraction may be performed on the noise removed by the first-stage network by means of a signal compensation algorithm to extract signals that belong to valid information in the noise removed by the first-stage network. A method of signal extraction may be as follows:
Method 1: multiplying the noise removed by the first-stage network by a set coefficient to obtain signals that belong to valid information in the noise removed by the first-stage network;
Method 2: performing low-frequency domain extraction processing on the noise removed by the first-stage network to obtain signals that belong to valid information in the noise removed by the first-stage network; or
Method 3: inputting the noise removed by the first-stage network into a signal extraction network obtained in a deep learning method to obtain signals that belong to valid information in the noise removed by the first-stage network.

The noise signals removed by the first-stage network in a determined proportion are first added to training input signal data carrying an absolute noise level of 300 corresponding to the second-stage network, and then the training input signal data are input into the second-stage network to obtain an output result of the second-stage network. A loss value of the second-stage network may be obtained based on a difference in noise level between the output result of the second-stage network and training target signal data carrying an absolute noise level of 200 corresponding to the second-stage network. Subsequently, individual gradient descent and joint gradient descent may be performed based on the loss value of the second-stage network.

In the present embodiment, between training of two adjacent stages of networks, a part of noise signals removed by a previous-stage network are added to training input signal data corresponding to a current-stage network, so as to ensure that signals are not lost excessively in a stage-by-stage denoising training process, thereby achieving an objective of ensuring denoising under a better signal-to-noise ratio condition.

In some embodiments, the multiple stages of the network are networks with a same network structure, or the multiple stages of the network are networks with different network structures.

The multi-stage network may be of a same network structure, or different network structures may be used based on requirements.

In some embodiments, when the multiple stages of the network are networks with different network structures, a previous-stage network in two adjacent stages of networks has higher structural complexity than a subsequent-stage network, and/or the previous-stage network in the two adjacent stages of networks has a larger number of parameters than the subsequent-stage network.

Description is made by taking two adjacent stages of networks, i.e., a first-stage network and a second-stage network, as an example.

The first-stage network is closer to the input end of the model than the second-stage network. The first-stage network faces larger noise and has relatively high requirements on a signal-to-noise ratio maintaining capability of the first-stage network. Therefore, the first-stage network may have higher structural complexity, and/or the first-stage network may have a larger number of parameters. However, the second-stage network faces smaller noise and has relatively low requirements on a signal-to-noise ratio maintaining capability of the second-stage network and relatively high requirements on a signal restoring capability of the second-stage network. Therefore, the second-stage network may have lower structural complexity, and/or the second-stage network may have a smaller number of parameters.

In some embodiments, the signal data includes at least one of CT raw data or CT images.

In a signal such as a CT image, a structure in the CT image belongs to valid information. Therefore, the signals extracted from the noise removed by the previous-stage network may include structure information in the CT image.

In some embodiments, when the signal data includes CT raw data, and the CT raw data are CT multi-energy-level data, training input CT multi-energy-level data are input into the network in a combined manner or input into the network independently.

When the signal data includes CT images, and the CT images are CT multi-energy-level images, training input CT multi-energy-level images are input into the network in a combined manner or input into the network independently.

When the signal data includes CT raw data, and the CT raw data are CT multi-energy-level data, the training input signal data may be referred to as training input CT multi-energy-level data. The training input CT multi-energy-level data may be input into the network in a combined method. The combining method includes, but is not limited to: (1) combining the training input CT multi-energy-level data in a channel direction, and (2) combining the training input CT multi-energy-level data in an independent dimension, for example, combining the training input CT multi-energy-level data in a z direction. The training input CT multi-energy-level data may also be input into the network independently.

The training input CT multi-energy-level data are input into the network in a combined manner or input into the network independently., and loss values of the training input CT multi-energy-level data are trained together subsequently.

When the signal data includes CT images, and the CT images are CT multi-energy-level images, the training input signal data may be referred to as training input CT multi-energy-level images. The training input CT multi-energy-level images may be input into the network in a combined method. The combining method includes, but is not limited to: (1) combining the training input CT multi-energy-level images in a channel direction, and (2) combining the training input CT multi-energy-level images in an independent dimension, for example, combining the training input CT multi-energy-level images in a z direction. The training input CT multi-energy-level images may also be input into the network independently.

The training input CT multi-energy-level images are input into the network in a combined manner or input into the network independently., and loss values of the training input CT multi-energy-level images are trained together subsequently.

For better understanding of the above method for training a signal denoising model, an application embodiment is described in detail below. In the application embodiment, the signal denoising model includes a four-stage network, and a denoising calibration range is divided by an absolute noise level. It is assumed that a denoising calibration range corresponding to a first-stage network is 400 to 300, a denoising calibration range corresponding to a second-stage network is 300 to 200, a denoising calibration range corresponding to a third-stage network is 200 to 100, and a denoising calibration range corresponding to a fourth-stage network is 100 to 0.

Training signal data corresponding to each stage network is obtained based on a denoising calibration range corresponding to each stage network. Each piece of training signal data includes training input signal data and training target signal data. A noise level carried in the training input signal data is an upper limit value of the denoising calibration range corresponding to a respective stage network, and a noise level carried in the training target signal data is a lower limit value of the denoising calibration range corresponding to the respective stage network.

The training input signal data corresponding to the first-stage network is denoted as signal-input (400), and the training target signal data corresponding to the first-stage network is denoted as signal-target (300). The training input signal data corresponding to the second-stage network is denoted as signal-input (300), and the training target signal data corresponding to the second-stage network is denoted as signal-target (200). The training input signal data corresponding to the third-stage network is denoted as signal-input (200), and the training target signal data corresponding to the third-stage network is denoted as signal-target (100). The training input signal data corresponding to the fourth-stage network is denoted as signal-input (100), and the training target signal data corresponding to the fourth-stage network is denoted as signal-target (0).

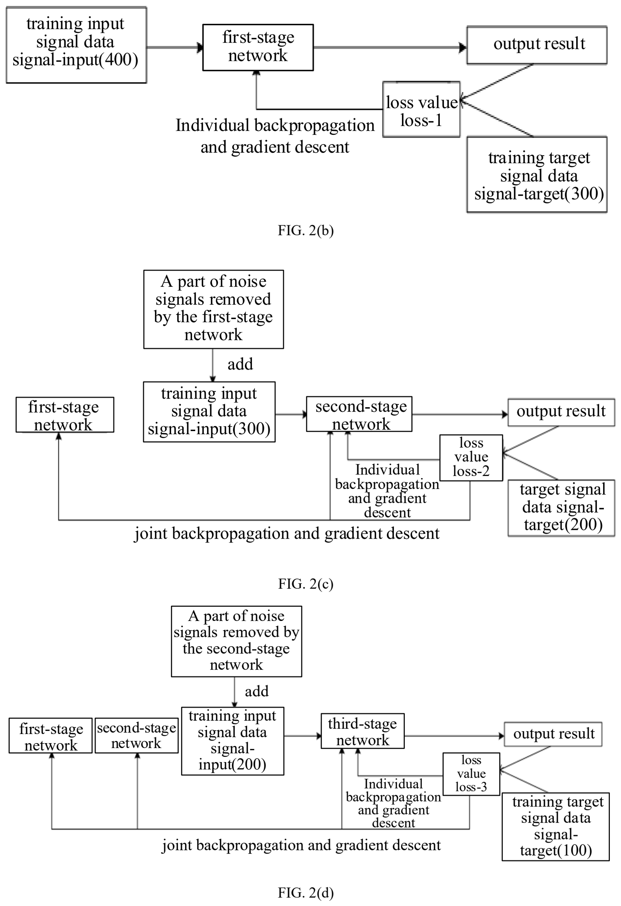

During first-stage denoising training, as shown in FIG. 2(b), the training input signal data signal-input (400) corresponding to the first-stage network may be input into the first-stage network. A loss function is configured to calculate a difference in noise level between an output result of the first-stage network and the training target signal data signal-target (300) corresponding to the first-stage network, so as to obtain a loss value loss-1 of the first-stage network. Based on the loss value loss-1 of the first-stage network, backpropagation and gradient descent are performed individually on the first-stage network, so as to complete the first-stage denoising training.

After the first-stage denoising training is completed, second-stage denoising training is performed. As shown in FIG. 2(c), the noise signals removed by the first-stage network may be added to the training input signal data signal-input (300) corresponding to the second-stage network, and then the training input signal data signal-input (300) are input into the second-stage network. A loss function is configured to calculate a difference in noise level between an output result of the second-stage network and the training target signal data signal-target (200) corresponding to the second-stage network, so as to obtain a loss value loss-2 of the second-stage network. Based on the loss value loss-2 of the second-stage network, backpropagation and gradient descent are performed individually on the second-stage network. After the backpropagation and gradient descent are performed individually, backpropagation and gradient descent are further performed on the first-stage network and the second-stage network jointly based on the loss value loss-2 of the second-stage network, so as to complete the second-stage denoising training.

After the second-stage denoising training is completed, third-stage denoising training is performed. As shown in FIG. 2(d), the noise signals removed by the second-stage network may be added to the training input signal data signal-input (200) corresponding to the third-stage network, and then the training input signal data signal-input (200) are input into the third-stage network. A loss function is configured to calculate a difference in noise level between an output result of the third-stage network and the training target signal data signal-target (100) corresponding to the third-stage network, so as to obtain a loss value loss-3 of the third-stage network. Based on the loss value loss-3 of the third-stage network, backpropagation and gradient descent are performed individually on the third-stage network. After the backpropagation and gradient descent are performed individually, backpropagation and gradient descent are further performed on the first-stage network, the second-stage network, and the third-stage network jointly based on the loss value loss-3 of the third-stage network, so as to complete the third-stage denoising training.

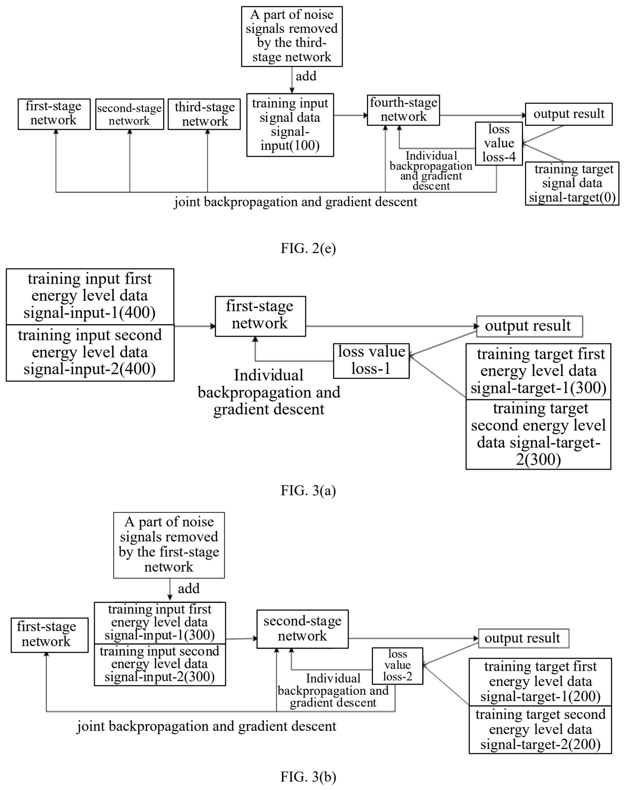

After the third-stage denoising training is completed, fourth-stage denoising training is performed. As shown in FIG. 2(e), the noise signals removed by the third-stage network may be added to the training input signal data signal-input (100) corresponding to the fourth-stage network, and then the training input signal data signal-input (100) are input into the fourth-stage network. A loss function is configured to calculate a difference in noise level between an output result of the fourth-stage network and the training target signal data signal-target (0) corresponding to the fourth-stage network, so as to obtain a loss value loss-4 of the fourth-stage network. Based on the loss value loss-4 of the fourth-stage network, backpropagation and gradient descent are performed individually on the fourth-stage network. After the backpropagation and gradient descent are performed individually, backpropagation and gradient descent are further performed on the first-stage network, the second-stage network, the third-stage network, and the fourth-stage network jointly based on the loss value loss-4 of the fourth-stage network, so as to complete the fourth-stage denoising training.

After the fourth-stage denoising training is completed, one iteration may be deemed to be completed. Two or more iterations may be performed based on actual conditions. After the iterations are completed, a signal denoising model is obtained.

When the signal data are CT multi-energy-level data and the number of energy levels is two, at this time, a schematic diagram of a configuration of first-stage denoising training is as shown in FIG. 3(a), and a schematic diagram of a configuration of second-stage denoising training is as shown in FIG. 3(b). A schematic diagram of a configuration of third-stage denoising training and a schematic diagram of a configuration of fourth-stage denoising training are not shown.

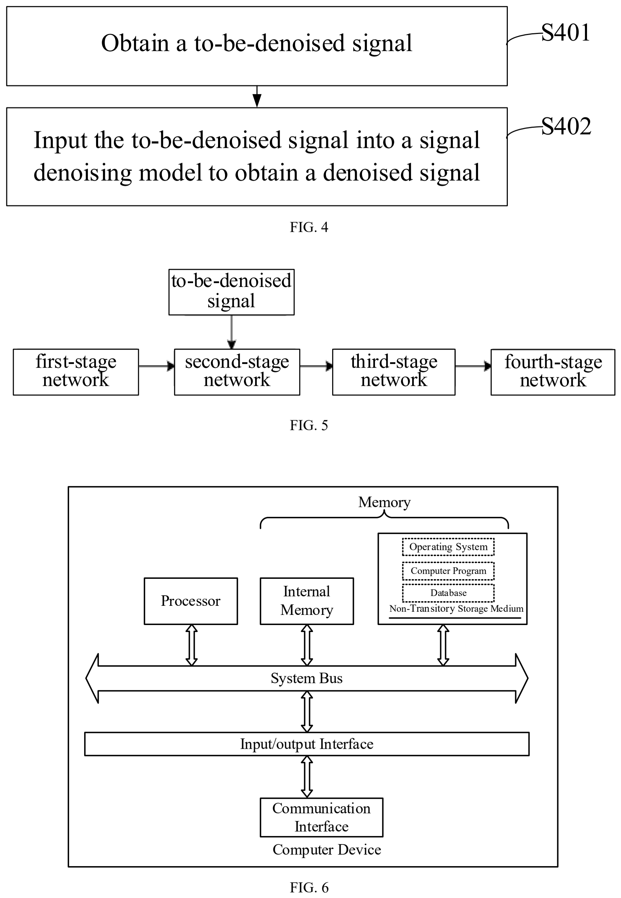

The signal denoising method provided in the present application belongs to an inference phase. The signal denoising method may be executed by a computer device, and may include steps shown in FIG. 4.

In step S401, a to-be-denoised signal is obtained.

When a signal in a training phase is CT raw data, to-be-denoised CT raw data may be obtained in an inference phase. When a signal in the training phase is a CT image, a to-be-denoised CT image may be obtained in the inference phase.

In step S402, the to-be-denoised signal is input into a signal denoising model to obtain a denoised signal.

The signal denoising model is trained by using the method for training a signal denoising model described in the above-mentioned embodiment.

After the to-be-denoised signal is obtained, the to-be-denoised signal may be input into the signal denoising model, and a denoised signal is obtained based on an output result of the signal denoising model.

The signal denoising model adopted in the present embodiment is obtained by means of a stage-by-stage denoising training method. In a training process, gradient descent is performed not only on a single-stage network individually but also on a multi-stage network jointly, which can improve denoising capability of the signal denoising model. In addition, a part of noise signals removed by a previous-stage network of a current-stage network are added to training signal data corresponding to the current-stage network to compensate and restore signals, so as to avoid a situation that signals are weakened after denoising by each stage network in the training process, so that the signal denoising model can perform denoising while ensuring signal accuracy. Therefore, the obtained denoised signal contains relatively high signal accuracy and retains many signals.

In some embodiments, inputting the to-be-denoised signal into the signal denoising model to obtain a denoised signal includes: determining a noise level of the to-be-denoised signal, determining a target input network in the signal denoising model based on the noise level of the to-be-denoised signal, where the noise level of the to-be-denoised signal is within a denoising calibration range of the target input network, and inputting the to-be-denoised signal into the target input network, so that forward multiple stages of the network starting from the target input network in the signal denoising model perform stage-by-stage denoising processing on the to-be-denoised signal to obtain the denoised signal.

A forward direction (which may also be referred to as a forward direction) is a direction from an input end of the model to an output end of the model. Correspondingly, a backward direction (which may also be referred to as a reverse direction) is a direction from the output end of the model to the input end of the model.

A denoising calibration range is divided by an absolute noise level. The signal denoising model includes a four-stage network. For example, a denoising calibration range corresponding to a first-stage network is 400 to 300, a denoising calibration range corresponding to a second-stage network is 300 to 200, a denoising calibration range corresponding to a third-stage network is 200 to 100, and a denoising calibration range corresponding to a fourth-stage network is 100 to 0.

When a noise level of a to-be-denoised signal is 250, and 250 is within a denoising calibration range of 300 to 200, the second-stage network may be used as a target input network. Forward multiple stages of the network starting from the second-stage network include a second-stage network, a third-stage network, and a fourth-stage network. As shown in FIG. 5, after the to-be-denoised signal is input into the second-stage network, stage-by-stage denoising processing is performed on the to-be-denoised signal by means of the second-stage network, the third-stage network, and the fourth-stage network, and a denoised signal is obtained based on an output result of the fourth-stage network. The noise level of the to-be-denoised signal may be estimated by means of a noise extraction algorithm.

A denoising calibration range is divided by a relative noise level. The signal denoising model includes a four-stage network. For example, a denoising calibration range corresponding to a first-stage network is 100% to 75%, a denoising calibration range corresponding to a second-stage network is 75% to 50%, a denoising calibration range corresponding to a third-stage network is 50% to 25%, and a denoising calibration range corresponding to a fourth-stage network is 25% to 0.

When a calibrated noise value is 500 and a noise level of a to-be-denoised signal is 300, a relative noise level of the to-be-denoised signal relative to the calibrated noise value is 60%. 60% is within a denoising calibration range of 75% to 50%, so that the second-stage network may be used as a target input network. Forward multiple stages of the network starting from the second-stage network include a second-stage network, a third-stage network, and a fourth-stage network. As shown in FIG. 5, after the to-be-denoised signal is input into the second-stage network, stage-by-stage denoising processing is performed on the to-be-denoised signal by means of the second-stage network, the third-stage network, and the fourth-stage network, and a denoised signal is obtained based on an output result of the fourth-stage network. The noise level of the to-be-denoised signal may be estimated by means of a noise extraction algorithm.

In the present embodiment, during inference, the stage network from which to input the to-be-denoised signal can be dynamically determined based on a noise level of the to-be-denoised signal, so that a result can be obtained more quickly and denoising efficiency is improved.

In an embodiment, the present application further provides a medical image denoising method, comprising: obtaining medical data; determining a noise level of the medical data; inputting the medical data into a multi-stage denoising network based on the noise level of the medical data to perform stage-by-stage denoising processing, thereby obtaining denoised medical data. The medical data includes medical scan data or medical images, such as CT raw data or CT images.

The multi-stage denoising network includes at least two denoising network modules. In two adjacent denoising network modules, an output of a previous denoising network module is an input of a next denoising network module, and each denoising network module is configured to remove noise in a different range. The multi-stage denoising network is obtained by training according to any one of the signal denoising model training methods described in the above embodiments.

In an embodiment, a target input denoising network module is determined in the multi-stage denoising network based on the noise level of the medical data, and then the medical data is input into the target input denoising network module to perform stage-by-stage denoising processing, thereby obtaining the denoised medical data. The noise level of the medical data falls within a denoising calibration range of the target input denoising network module. Specific details can refer to the description of similar embodiments above.

In an embodiment, in two adjacent stages of denoising processing, a predetermined proportion of noise signals removed by the previous denoising network module is added to the input of the next denoising network module, so as to compensate for and restore the signals, avoiding the situation where the signal is weakened after denoising by each stage network during training, thereby enabling the signal denoising model to perform denoising while ensuring the accuracy of the signals.

In an embodiment, the multi-stage denoising network is obtained through multi-stage training. The multi-stage training includes: performing independent pre-training on at least two denoising network modules in the multi-stage denoising network respectively to obtain an initial multi-stage denoising network; after a non-first-stage denoising network module completes the independent pre-training, performing joint training on a plurality of denoising network modules that have been independently pre-trained to update the initial multi-stage denoising network.

For example, the independent pre-training may refer to the individual gradient descent, and the joint training may refer to the joint gradient descent. The multi-stage denoising network of the present application adopts a staged training strategy. For example, first, the independent pre-training is performed on the first-stage and second-stage denoising networks to obtain a stable initial model. Then, the parameters of all denoising networks are unfrozen, and end-to-end joint training is performed to optimize the overall performance of the entire denoising network. Specific details of the staged training process can refer to the description of the relevant embodiments above.

It can be understood that other implementation details of the medical image denoising method can be found in the relevant embodiments above, and corresponding technical effects are achieved.

In an embodiment, the present application further provides a medical image denoising method based on a multi-stage denoising network. The multi-stage denoising network includes at least two denoising network modules connected in series. In two adjacent denoising network modules, an output of a previous denoising network module is an input of a next denoising network module. The method includes: obtaining medical data; determining a noise level of the medical data; selecting, from the multi-stage denoising network, a denoising network module as a target first-stage denoising network module based on the noise level of the medical data; inputting the medical data into the target first-stage denoising network module to perform stage-by-stage denoising processing, thereby obtaining denoised medical data. The medical data includes medical scan data or medical images.

The medical image denoising method can adaptively select from which stage denoising network to input. For example, it can be determined into which denoising calibration range corresponding to a denoising network module the noise level of the medical data falls, and then the denoising network module corresponding to the denoising calibration range into which the noise level of the medical data falls is taken as the target first-stage denoising network module.

It can be understood that other implementation details of the medical image denoising method based on a multi-stage denoising network can be found in the relevant embodiments above, and corresponding technical effects are achieved.

It should be understood that although all steps in the flowcharts corresponding to the above-mentioned embodiments are displayed in sequence as indicated by arrows, these steps are not necessarily executed in sequence as indicated by the arrows. Unless explicitly specified in the present application, execution of these steps is not strictly limited, and these steps may be executed in other sequences. Moreover, at least some steps in the flowcharts corresponding to the above-mentioned embodiments may include a plurality of steps or stages. These steps or stages are not necessarily executed at the same time, but may be executed at different times. These steps or stages are not necessarily executed in sequence, but may be executed alternately or in turn with at least some of other steps or steps in other stages.

In the present application, an apparatus for training a signal denoising model is provided, including: a model acquisition module configured to perform stage-by-stage denoising training on a multi-stage network based on training signal data corresponding to the multi-stage network, to obtain a signal denoising model; and a training module configured to obtain, when a current stage subjected to the stage-by-stage denoising training is not a first stage, a loss value of a current-stage network based on training signal data corresponding to the current-stage network, perform gradient descent on the current-stage network individually based on the loss value, and perform, based on the loss value, gradient descent jointly on current multiple stages of the network that have undergone the gradient descent individually.

In some embodiments, the training signal data corresponding to the current-stage network are added with a part of noise signals removed by a previous-stage network of the current-stage network.

In some embodiments, the training module is further configured to perform layer-by-layer backward calculation on the current-stage network based on the loss value to obtain a first type of gradient values, and perform gradient descent on the current-stage network based on the first type of gradient values.

In some embodiments, the training module is further configured to perform, based on the loss value, network-by-network backward calculation on the current multiple stages of the network that have undergone the gradient descent individually to obtain a second type of gradient values, and perform, based on the second type of gradient values, gradient descent on the current multiple stages of the network that have undergone the gradient descent individually.

In some embodiments, the apparatus for training a signal denoising model further includes a sample acquisition module configured to obtain training signal data corresponding to each stage network based on a denoising calibration range corresponding to each stage network, where each piece of training signal data includes training input signal data and training target signal data, a noise level carried in the training input signal data is an upper limit value of the denoising calibration range corresponding to a respective stage network, and a noise level carried in the training target signal data is a lower limit value of the denoising calibration range corresponding to the respective stage network.

The training module is further configured to input the training input signal data corresponding to the current-stage network into the current-stage network, and obtain the loss value of the current-stage network based on a difference in noise level between an output result of the current-stage network and the training target signal data corresponding to the current-stage network.

In some embodiments, the training module is further configured to obtain noise removed by a previous-stage network of the current-stage network based on training input signal data and training target signal data corresponding to the previous-stage network, perform signal extraction on the noise removed by the previous-stage network of the current-stage network, and add a part of extracted signals to the training input signal data corresponding to the current-stage network.

In some embodiments, the multiple stages of the network are networks with an identical network structure, or the multiple stages of the network are networks with different network structures.

In some embodiments, when the multiple stages of the network are networks with different network structures, a previous-stage network in two adjacent stages of networks has a higher structural complexity than a subsequent-stage network, and/or the previous-stage network in the two adjacent stages of networks has a larger parameter quantity than the subsequent-stage network.

In some embodiments, the signal data includes at least one of CT raw data or CT images.

In some embodiments, when the signal data includes CT raw data, and the CT raw data are CT multi-energy-level data, the training input CT multi-energy-level data are input into the network in a combined manner or input into the network independently; and when the signal data includes CT images, and the CT images are CT multi-energy-level images, the training input CT multi-energy-level images are input into the network in a combined manner or input into the network independently.

In the present application, a signal denoising apparatus is provided, including: a signal acquisition module configured to obtain a to-be-denoised signal, and a denoising module configured to input the to-be-denoised signal into a signal denoising model to obtain a denoised signal, where the signal denoising model is trained by using the method for training a signal denoising model described in the above-mentioned embodiments.

In some embodiments, the denoising module is further configured to determine a noise level of the to-be-denoised signal, determine a target input network in the signal denoising model based on the noise level of the to-be-denoised signal, where the noise level of the to-be-denoised signal is within a denoising calibration range of the target input network, and input the to-be-denoised signal into the target input network, so that forward multiple stages of the network starting from the target input network in the signal denoising model perform stage-by-stage denoising processing on the to-be-denoised signal to obtain the denoised signal.

In an exemplary embodiment, a computer device is provided, a schematic diagram of an internal configuration of which may be shown in FIG. 6. The computer device includes a processor, a memory, an input/output (I/O) interface, and a communication interface. The processor, the memory, and the input/output interface are connected through a system bus, and the communication interface is connected to the system bus through the input/output interface. The processor of the computer device is configured to provide computing and control capabilities. The memory of the computer device includes a non-transitory storage medium and an internal memory. The non-transitory storage medium stores an operating system, a computer program, and a database. The internal memory provides an environment for running the operating system and the computer program in the non-transitory storage medium. The database of the computer device is configured to store data involved in the above-mentioned method. The input/output interface of the computer device is configured to exchange information between the processor and an external device. The communication interface of the computer device is configured to communicate with an external terminal through a network connection. The computer program, when executed by the processor, implements the method described in the above-mentioned embodiments.

In some embodiments, a computer device is provided, including a memory and a processor, the memory stores a computer program. The processor, when executing the computer program, implements the steps in the above-mentioned method embodiments.

In some embodiments, a computer-readable storage medium having a computer program stored thereon is provided. The computer program, when executed by a processor, causes the processor to implement the steps in the above-mentioned method embodiments.

In some embodiments, a computer program product having a computer program stored thereon is provided, the computer program, when executed by a processor, implements the steps in the above-mentioned method embodiments.

## Claims

1. A method for training a signal denoising model for medical image processing, comprising:
performing stage-by-stage denoising training on a multi-stage network based on training signal data corresponding to the multi-stage network, to obtain a signal denoising model (S101);
obtaining, when a current stage subjected to the stage-by-stage denoising training is not a first stage, a loss value of a current-stage network based on training signal data corresponding to the current-stage network (S102); and
after gradient descent is performed individually on the current-stage network based on the loss value, performing, based on the loss value, gradient descent jointly on current multiple stages of the network that have undergone the gradient descent individually (S102).

2. The method according to claim 1, wherein the training signal data corresponding to the current-stage network are added with a part of noise signals removed by a previous-stage network of the current-stage network;
optionally, a predetermined proportion of the noise signals removed by the previous-stage network of the current-stage network is added to the training signal data corresponding to the current-stage network, wherein predetermined proportions corresponding to all stages of denoising training are identical, or predetermined proportions corresponding to at least two stages of denoising training are different.

3. The method according to claim 1 or 2, wherein performing the gradient descent individually on the current-stage network based on the loss value comprises:
performing layer-by-layer backward calculation on the current-stage network based on the loss value to obtain a first type of gradient values; and
performing gradient descent on the current-stage network based on the first type of gradient values.

4. The method according to any one of claims 1 to 3, wherein performing, based on the loss value, gradient descent jointly on the current multiple stages of the network that have undergone the gradient descent individually comprises:
performing, based on the loss value, network-by-network backward calculation on the current multiple stages of the network that have undergone the gradient descent individually, to obtain a second type of gradient values; and
performing, based on the second type of gradient values, gradient descent on the current multiple stages of the network that have undergone the gradient descent individually.

5. The method according to any one of claims 1 to 4, wherein the method further comprises:
obtaining training signal data corresponding to each stage network based on a denoising calibration range corresponding to each stage network, each piece of training signal data comprising training input signal data and training target signal data, a noise level carried in the training input signal data being an upper limit value of the denoising calibration range corresponding to a respective stage network, and a noise level carried in the training target signal data being a lower limit value of the denoising calibration range corresponding to the respective stage network.

6. The method according to claim 5, wherein obtaining the loss value of the current-stage network based on the training signal data corresponding to the current-stage network comprises:
inputting the training input signal data corresponding to the current-stage network into the current-stage network; and
obtaining the loss value of the current-stage network based on a difference in noise level between an output result of the current-stage network and the training target signal data corresponding to the current-stage network.

7. The method according to claim 5, wherein before inputting the training input signal data corresponding to the current-stage network into the current-stage network, the method further comprises:
obtaining noise removed by the previous-stage network of the current-stage network based on training input signal data and training target signal data corresponding to the previous-stage network;
performing signal extraction on the noise removed by the previous-stage network of the current-stage network; and
adding a part of extracted signals to the training input signal data corresponding to the current-stage network.

8. The method according to claim 7, wherein obtaining the noise removed by the previous-stage network of the current-stage network based on the training input signal data and the training target signal data corresponding to the previous-stage network comprises:
taking difference signal data between the training input signal data and the training target signal data corresponding to the previous-stage network as the noise removed by the previous-stage network of the current-stage network.

9. The method according to any one of claims 1 to 8, wherein the signal data comprises at least one of CT raw data or CT images;
optionally, when the signal data comprises CT raw data and the CT raw data are CT multi-energy-level data, training input CT multi-energy-level data are input into the network in a combined manner or input into the network independently; and
optionally, when the signal data comprises CT images and the CT images are CT multi-energy-level images, training input CT multi-energy-level images are input into the network in a combined manner or input into the network independently.

10. A medical image denoising method, comprising:
obtaining medical data, wherein the medical data comprises medical scan data or medical images;
determining a noise level of the medical data; and
inputting the medical data into a multi-stage denoising network based on the noise level of the medical data to perform stage-by-stage denoising processing to obtain denoised medical data, wherein the multi-stage denoising network comprises at least two denoising network modules, an output of a previous denoising network module is an input of a next denoising network module in two adjacent denoising network modules, and each denoising network module is configured to remove noise in a different range; wherein the multi-stage denoising network is trained by using the method for training the signal denoising model according to any one of claims 1 to 9.

11. The medical image denoising method according to claim 10, wherein inputting the medical data into the multi-stage denoising network based on the noise level of the medical data to perform stage-by-stage denoising processing to obtain the denoised medical data comprises:
determining a target input denoising network module in the multi-stage denoising network based on the noise level of the medical data; wherein the noise level of the medical data falls within a denoising calibration range of the target input denoising network module; and
inputting the medical data into the target input denoising network module to perform stage-by-stage denoising processing to obtain the denoised medical data.

12. The medical image denoising method according to claim 10, wherein in two adjacent stages of denoising processing, a predetermined proportion of noise signals removed by the previous denoising network module is added to the input of the next denoising network module.

13. The medical image denoising method according to claim 10, wherein the multi-stage denoising network is obtained through multi-stage training, and the multi-stage training comprises:
performing independent pre-training on at least two denoising network modules in the multi-stage denoising network respectively to obtain an initial multi-stage denoising network; and
after a non-first-stage denoising network module completes the independent pre-training, performing joint training on a plurality of denoising network modules that have been independently pre-trained to update the initial multi-stage denoising network.

14. An apparatus for training a signal denoising model for medical image processing, comprising:
a model acquisition module configured to perform stage-by-stage denoising training on a multi-stage network based on training signal data corresponding to the multi-stage network, to obtain a signal denoising model; and
a training module configured to obtain, when a current stage subjected to the stage-by-stage denoising training is not a first stage, a loss value of a current-stage network based on training signal data corresponding to the current-stage network, and after gradient descent is performed individually on the current-stage network based on the loss value, perform, based on the loss value, gradient descent jointly on current multiple stages of the network that have undergone the gradient descent individually.

15. A computer device, comprising a memory and a processor, wherein the memory stores a computer program, and the processor, when executing the computer program, implements the method according to any one of claims 1 to 13.
